# EUROPÄISCHE PATENTANMELDUNG

(11) **EP 1 547 532 A2**
(43) Veröffentlichungstag der Anmeldung: **29.06.2005**
(21) Anmeldenummer: 04029042.1
(22) Anmeldetag: 08.12.2004
(51) Int. Cl.: A61B 17/16

(54) **Osteotom zur schonenden Schleimhautanhebung**

(30) Priorität: 17.12.2003 DE 10359304
(71) Anmelder: Bauer, Florian, 84036 Landshut (DE)
(72) Erfinder: Bauer, Florian, 84036 Landshut (DE)

(57) **Zusammenfassung**

Osteotom, welches zur Schaffung eines Zuganges zur Kieferhöhle und einer Anhebung der Kieferhöhlenschleimhaut vorgesehen ist. Mit einem Mandrin mit definiertem Überstand am distalen Ende und einer Justierhülse für das distale Ende lässt sich die kieferhöhlenseitige Kompakta mit einem Schlag auf das proximale Ende des Mandrin kontrolliert frakturieren. Mit dem dazugehörigen Ballonkatheter lässt sich dann die Kieferhöhlenschleimhaut im Sinne eines Sinusliftes gewebeschonend anheben.

## Beschreibung

Gegenstand des Patents sind chirurgische Instrumente, die insbesondere bei der Durchführung von Sinusbodenelevationen eingesetzt werden können.
In vielen Fällen der Implantation im Oberkiefer-Seitenzahnbereich ist der ortständige Knochen wegen der hier wirksamen zentripetalen und zentrifugalen Resorptionsprozesse zu schwach, um ein für diese Region ausreichend dimensioniertes Implantat aufzunehmen.

Die operative Vermehrung des Knochens, die sog. Augmentation des Kieferhöhlenbodens, setzt eine Elevation der so genannten SCHNEIDER-Membran in der Kieferhöhle voraus. Bei der Abhebung sollte diese Schleimhaut aus verschiedenen Gründen nicht verletzt werden. Als Zugang zur Schleimhaut bieten sich prinzipiell zwei Möglichkeiten an:
1. Das Anlegen eines Knochenfensters (sog. TATUM-window) in der unteren Kieferhöhlen -Seitenwand wird als direkter, weil unter Sicht des Auges stattfindender Lift, bezeichnet. (Spiekermann, H., Donath, K., Jovanovic, S., and Richter, J., "Zukunftsperspektiven," *Implantologie* Farbatlanten der Zahnmedizin ed. Rateitschak,K.H.; Wolf,H.F.; 1996, pp. 356-367);
   ( Tatum, O.H.Jr. (1986): Maxillary and sinus implant reconstructions. Dent Clin North Am, 30(2):207-229).
2. Das Vortreiben eines Osteotoms vom Kieferkamm aus (Sinusbodenelevation nach SUMMERS) wird als indirekte Elevation bezeichnet. Sie kann eine vorherige Trepanfräsung des Kieferkamms notwendig machen. Das Ausmaß einer dieserart durchgeführten Schleimhautelevation ist mit den bisher benutzten Instrumentarien deutlich eingeschränkt, da die Anhebung 2-3 mm nicht überschreiten sollte. Außerdem wird teilweise in der Literatur bei dieser Technik zur Sicherheit eine gleichzeitige endoskopische Kontrolle der Kieferhöhle vorgeschlagen, was einen Zweiteingriff mit nicht unerheblichem apparativen Mehraufwand bedeutet. (Summers, R.B. (1994): A new concept in maxillary implant surgery: The osteotome technique. Compend Contin Educ Dent XV(2):152-160); (Summers, R.B. (1994): The osteotome technique: Part 2 - The ridge expansion osteotomy (REO). Compend Contin Educ Dent XV(4):422-434); (Summers, R.B. (1994): The osteotome technique: Part 3 - Less invasing method of elevating the sinus floor. Compend Contin Educ Dent XV(6):698-708).

Bereits Anfang der 90er Jahre schlug Prof.Dr. K.-U. Benner, Ludwig-Maximilians-Universität München, durch mündlichen Vortrag in Vorlesungen und Symposien vor, zur Elevation der Schleimhaut einen aufblasbaren Ballon einzusetzen.

Bekannt ist - Gebrauchsmuster DE 296 06 335 U1 - ein Osteotom, das als Implantatbettformer verwendet wird. Dieses Instrument dient zur operativen Aufspaltung und Spreizung von Knochenabschnitten zum Einsetzen von im Querschnitt kreisförmigen Implantaten, insbesondere im Oberkiefer. Das Osteotom in der Entgegenhaltung schafft ein Implantatbett durch Expansion und Knochenkompression des Knochenmaterials einer im Querschnitt kleineren Öffnung im Knochen als dieses Osteotom. Diese Intervention wird mit im Querschnitt immer größer werdenden Osteotomen durchgeführt, bis etwa der Implantatquerschnitt erreicht ist. Das Knochenmaterial entspannt sich nach dem Herausziehen des Dentalinstrumentes und dem Einsetzen des Implantates und hält nun dieses fest.
Der Sinn dieses Instrumentes besteht allein in der Kondensierung des ortständigen Knochens.
Dieses Osteotom hat mit dem Erfindungsgemäßen schon deshalb keine Gemeinsamkeiten, weil es zu dessen Brech- und Schutzfunktion keinerlei Hinweise gibt.
Weiter bekannt ist ein Osteotom gemäß der Europäischen Patentanmeldung EP 1 269 933 A2.
Die Ansprüche 1 bis 10 und 29 haben mit der gegenständlichen Erfindung nichts gemeinsam, da insoweit ein Bohrer geschützt ist. Das jeweilige Osteotom gemäß der Ansprüche 11 bis 25 unterscheidet sich vom Anspruch 1 der Erfindung, auf den alle weiteren Ansprüche unmittelbar oder mittelbar rückbezogen sind, dadurch, dass nur die Erfindung am distalen Ende eine verstellbare Hülse aufweist, die als Anschlag zur Justierung der Länge des distalen Endes dient. Die Patentansprüche 2 bis 10 entfernen sich dann inhaltlich durch den unterschiedlichen Therapieeinsatz von dem der Entgegenhaltung. Während alle Ansprüche der Entgegenhaltung darauf abzielen, den mit Augmentatmaterial gefüllten Ballon in der Kieferhöhle unter der Kieferhöhlenschleimhaut zu belassen, dienen die Osteotome gemäß den Patentansprüchen dazu, einen Ballon nur zur schonenden Abhebung der Kieferhöhlenschleimhaut zu verwenden und ihn dann unmittelbar nach Gebrauch wieder zu entfernen.

Insbesondere für diese Methode wurde das gegenständliche Instrumentarium entwickelt. Es besteht aus:
1. Einem z.B. metallischen Osteotom mit Mandrin.
2. Das Mandrin überragt, vollständig in das Osteotom eingeführt, dieses leicht.
3. Einem Katheter, der den herausziehbaren Mandrin im Osteotom ersetzen kann. An der Spitze des Katheters befindet sich ein aufblasbarer Ballon. Katheter und Ballon sind über einen Y-Anschluss entlüftbar und mit einer Flüssigkeit befüllbar

Das entwickelte Osteotom weist eine Vielzahl von Vorteilen auf.
Durch eine Fase am distalen Ende des Osteotoms kann dieses definiert in die durch einen Hohlzylinderbohrer angefräste kieferhöhlenseitige Kompakta positioniert werden. Desweiteren kann diese Fase als Schutz des Ballons vor scharfen Knochenkanten dienen.
Eine sich am distalen Ende befindliche Hülse kann nach Platzierung des Osteotoms an der kieferhöhlenseitigen Kompakta bis zum Anschlag an die Schleimhaut, bzw. an die mundhöhlenseitige Kompakta verschoben und fixiert werden, um ein unbeabsichtigtes Eindringen des Osteotoms in die Kieferhöhle zu verhindern.
Nach Einführen des Mandrins in das Osteotom steht der Mandrin in definierter Länge am proximalen Ende heraus. Durch einen Schlag auf das proximale Ende des Mandrins lässt sich die kieferhöhlenseitige Kompakta kontrolliert frakturieren.
Nach diesem Arbeitsschritt wird das Mandrin gegen den Ballonkatheter ausgetauscht. Dieser ist vor dem Einsetzen mit einem inkompressiblen Medium gefüllt worden. Nach Ablassen der sich im Ballonkatheter befindlichen Luftsäule durch das Y-Stück wird an diesem ein Ventil geschlossen. Anschließend wird der Ballonkatheter mit einem definierbaren Volumen befüllt. Die Kieferhöhlenschleimhaut wird abgehoben. Der entstandene Hohlraum kann mit Hilfe von handelsüblichen Instrumenten und mit entsprechenden Knochenaufbaumaterialien ausgefüllt werden.

Die ballonassistierte Schleimhautelevation (BASE) hat gegenüber der indirekten und auch der direkten Schleimhautelevation mit den bisher zur Verfügung stehenden Instrumenten folgende Vorteile:
- Sie ist sicher. Eine zusätzliche endoskopische Kontrolle ist nicht nötig. Auch besteht bei den nicht ganz seltenen sog. UNDERWOOD-Septen keine Gefahr des Schleimhauteinrisses. Dieser ist typisch bei Ablösung mit herkömmlichem Instrumentarium wegen der meist messerscharfen Kanten dieser Septen.
- Sie ermöglicht eine gute Einschätzung des entstandenen Raumes. Der Operateur erhält den quantitativen Bedarf an Knochenaufbaumaterial durch Vergleich mit dem zur Befüllung des Ballons verwendeten Flüssigkeitsvolumens.
- Sie ist schnell und weniger traumatisch. Das Aufblasen des Ballons dauert ca. 2 - 3 Sekunden, die Exposition des Operations-Situs kann damit deutlich verkürzt werden.
- Sie ist raumgreifend. Sie gestattet auch und gerade bei der Sinusbodenelevation eine sichere Anhebung der Bodenschleimhaut um weit mehr als die sonst möglichen 2 bis 3 mm.

Diese einfachere und schnellere Elevationstechnik mit diesem Instrumentarium lässt sich also in allen Fällen eines Sinuslifts (ob nach Bildung eines TATUM-Fensters oder im Rahmen des SUMMERS-Lifts) mit den beschriebenen Vorteilen einsetzen.

## Patentansprüche

1. Osteotom mit einem Hohlzylinder, der zur Durchführung von Instrumenten geeignet ist, **dadurch gekennzeichnet, dass** es am distalen Ende eine verstellbare Hülse ausweist, die als Anschlag zur Justierung der Länge des distalen Endes dient.

2. Osteotom nach Anspruch 1, **dadurch gekennzeichnet, dass** es Rillen am distalen Ende ausweist, die als Hilfs- oder Orientierungsmarkierungen dienen.

3. Osteotom nach Anspruch 1 oder 2, **dadurch gekennzeichnet, dass** es eine Fase am distalen Ende aufweist.

4. Osteotom nach einem der Ansprüche 1 bis 3, **dadurch gekennzeichnet, dass** es einen zylindrischen Stab zum Einsatz in dem Hohlzylinder aufweist.

5. Osteotom nach Anspruch 4, **dadurch gekennzeichnet, dass** es einen Anschlag am proximalen Ende aufweist, vorzugsweise in Form eines Griffes.

6. Osteotom nach Anspruch 4 oder 5, **dadurch gekennzeichnet, dass** die Länge des Stabes so gewählt ist, dass der Stab in vollständig in den Hohlzylinder eingeführtem Zustand das distale Ende zwischen 0,1 und 5 mm, vorzugsweise zwischen 1 und 3 mm überragt.

7. Osteotom nach einem der Ansprüche eins bis drei, **dadurch gekennzeichnet, dass** es einen Katheter aufweist.

8. Osteotom nach Anspruch 7, **dadurch gekennzeichnet, dass** es ein vergößerbares distales Ende, vorzugsweise in Form eines Ballons, aufweist.

9. Osteotom nach Anspruch 7 oder 8, **dadurch gekennzeichnet, dass** es eine Befüllvorrichtung am proximalen Ende aufweist.

10. Osteotom nach einem der Ansprüche 7 bis 9, **dadurch gekennzeichnet, dass** die Befüllvorrichtung am proximalen Ende ein Y-Stück aufweist.
